# EUROPÄISCHE PATENTSCHRIFT

(11) **EP 2 880 459 B1**
(45) Veröffentlichungstag und Bekanntmachung des Hinweises auf die Patenterteilung: **09.09.2020**
(21) Anmeldenummer: 12774973.7
(22) Anmeldetag: 21.09.2012
(51) Int. Cl.: G01R 33/48, A61B 6/00

(54) **HYBRID-UNTERSUCHUNGSSYSTEM MIT EINEM MR-TOMOGRAPHEN, EINER RÖNTGENQUELLE UND EINEM RÖNTGENDETEKTOR**
HYBRID EXAMINATION SYSTEM HAVING AN MR SCANNER, AN X-RAY SOURCE AND AN X-RAY DETECTOR
SYSTÈME D'EXAMEN HYBRIDE DOTÉ D'UN IMAGEUR PAR RÉSONANCE MAGNÉTIQUE, D'UNE SOURCE DE RAYONS X ET D'UN DÉTECTEUR DE RAYONS X

(43) Veröffentlichungstag der Anmeldung: 10.06.2015
(73) Patentinhaber: Siemens Healthcare GmbH, 91052 Erlangen (DE)
(72) Erfinder: HEID, Oliver, 91052 Erlangen (DE); HELLER, Jürgen, 91080 Spardorf (DE); HUGHES, Timothy, OX12 7HA Wantage Oxfordshire (GB); KLEEMANN, Michael, 91054 Erlangen (DE)
(86) Internationale Anmeldenummer: PCT/EP2012/068596
(87) Internationale Veröffentlichungsnummer: WO 2014/044314

(56) Entgegenhaltungen:
- DE-A1-102008 045 276
- US-A1- 2003 123 612
- US-B1- 6 591 127

## Beschreibung

Die vorliegende Erfindung betrifft ein Untersuchungssystem gemäß Patentanspruch 1 sowie ein Verfahren zum Herstellen eines Angiogramms gemäß Patentanspruch 10.

Magentresonanztomographen zur bildgebenden Diagnostik für medizinische Zwecke sind aus dem Stand der Technik bekannt. Die Magnetresonanztomographie nutzt die Abhängigkeit der Relaxationszeiten angeregter Kernspins von der Umgebung der Atomkerne, um Informationen über eine räumliche Anordnung unterschiedlicher Gewebearten in einem Körper eines Patienten zu gewinnen. Magnetresonanztomographen eignen sich insbesondere zur kontraststarken Abbildung von Weichteilen.

Röntgengeräte zur bildgebenden Diagnostik für medizinische Zwecke sind ebenfalls aus dem Stand der Technik bekannt. Die Radiografie nutzt die unterschiedliche Durchlässigkeit unterschiedlicher Gewebearten für Röntgenstrahlung zur Erzeugung einer Durchsicht eines Körperteils eines Patienten. Dabei ist es möglich, bewegte Bilder in Echtzeit zu erzeugen.

Die US 2008/0171931 A1 beschreibt ein Hybridsystem aus einem Magnetresonanztomographen und einem Röntgengerät, das es ermöglicht, mittels des Magnetresonanztomographen ein Bild eines Körperteils eines Patienten aufzunehmen und anschließend einen Eingriff am Körper des Patienten vorzunehmen, wobei eine optische Kontrolle mittels des Röntgengeräts erfolgt. Weitere Hybridsysteme aus einem MR-Tomographen und einem Röntgengerät sind aus US 2003/0123612 A1 und US 6,591,127 B1 bekannt.

Eine Aufgabe der vorliegenden Erfindung besteht darin, ein verbessertes Untersuchungssystem bereitzustellen. Diese Aufgabe wird durch ein Untersuchungssystem mit den Merkmalen des Anspruchs 1 gelöst. Eine weitere Aufgabe der vorliegenden Erfindung besteht darin, ein Verfahren zum Herstellen eines Angiogramms anzugeben. Diese Aufgabe wird durch ein Verfahren mit den Merkmalen des Anspruchs 10 gelöst. Bevorzugte Weiterbildungen sind in den abhängigen Ansprüchen angegeben.

Ein Untersuchungssystem umfasst einen Magnetresonanztomographen mit einer Zylinderspule zur Erzeugung eines Magnetfelds. Dabei umgibt die Zylinderspule einen Untersuchungsraum. In diesem Untersuchungsraum sind eine erste Röntgenquelle und ein erster Röntgendetektor angeordnet. Vorteilhafterweise erlaubt dieses Untersuchungssystem eine Gewinnung von Bildern mit hoher räumlicher Auflösung und gutem Weichteilekontrast mit den Methoden der Magnetresonanztomographie. Außerdem können mittels der ersten Röntgenquelle und des ersten Röntgendetektors Röntgenbilder mit hoher zeitlicher Auflösung erstellt werden. Die Integration des Magnetresonanztomographen mit dem aus Röntgenquelle und Röntgendetektor gebildeten Röntgensystem stellt sicher, dass mit beiden Methoden gewonnene anatomische Informationen zeitlich und räumlich konsistent sind. Die Kombination der beiden bildgebenden Verfahren liefert anatomische Informationen, die sich gegenseitig ergänzen, wodurch sich ein insgesamt erhältlicher Informationsgehalt erhöht. Dadurch verbessert sich vorteilhafterweise eine klinische Effektivität des Gesamtsystems.

In einer Ausführungsform des Untersuchungssystems sind die erste Röntgenquelle und der erste Röntgendetektor um eine in Längsrichtung des Untersuchungsraums angeordnete Drehachse drehbar. Vorteilhafterweise können mit dem aus Röntgenquelle und Röntgendetektor gebildeten Röntgensystem dann Röntgenaufnahmen aus unterschiedlichen Blickrichtungen erstellt werden.

In einer Ausführungsform des Untersuchungssystems sind die erste Röntgenquelle und der erste Röntgendetektor starr miteinander verbunden. Vorteilhafterweise wird dadurch sichergestellt, dass der Röntgendetektor bei einer Drehung von Röntgenquelle und Röntgendetektor um die Drehachse stets so angeordnet ist, dass er von der Röntgenquelle emittierte Röntgenstrahlung detektieren kann.

In einer Ausführungsform des Untersuchungssystems ist im Untersuchungsraum eine Gradientenspule mit einem ersten Gradientenspulenteil und einem zweiten Gradientenspulenteil angeordnet. Dabei sind der erste Gradientenspulenteil und der zweite Gradientenspulenteil in axiale Richtung beabstandet. Ferner sind die erste Röntgenquelle und der erste Röntgendetektor zwischen dem ersten Gradientenspulenteil und dem zweiten Gradientenspulenteil angeordnet. Vorteilhafterweise bildet sich bei dieser Anordnung im Zwischenraum zwischen dem ersten Gradientenspulenteil und dem zweiten Gradientenspulenteil ein Gradientenfeld heraus, das eine räumlich aufgelöste Untersuchung mittels Magnetresonanztomographie ermöglicht. Gleichzeitig ist das aus Röntgenquelle und Röntgendetektor gebildete Röntgensystem in diesem Zwischenraum angeordnet und erlaubt damit eine Erstellung von Röntgenaufnahmen des selben Bereichs, der auch mittels Magnetresonanztomographie dargestellt wird. Durch die Anordnung des Röntgensystems im Zwischenraum zwischen den Gradientenspulenteilen der Gradientenspule wird eine optische Behinderung oder Abschattung der Röntgenstrahlung durch die Gradientenspule vermieden.

Erfindungsgemäss ist eine Hochfrequenzspule im Untersuchungsraum angeordnet. In einer Ausführungsform ist diese zwischen dem ersten Gradientenspulenteil und dem zweiten Gradientenspulenteil angeordnet. Vorteilhafterweise kann die Hochfrequenzspule zum Aussenden hochfrequenter magnetischer Impulse und zum Empfangen von durch relaxierende Kernspins ausgesendeten Signale dienen. Die Anordnung der Hochfrequenzspule im Zwischenraum zwischen dem ersten Gradientenspulenteil und dem zweiten Gradientenspulenteil der Gradientenspule ermöglicht es, den selben im Untersuchungsraum angeordneten Teil eines Körpers eines Patienten bildgebend zu untersuchen, der auch durch das aus Röntgenquelle und Röntgendetektor gebildete Röntgensystem erfasst wird.

Erfindungsgemäss ist die Hochfrequenzspule starr mit der ersten Röntgenquelle verbunden. Vorteilhafterweise bleibt die gegenseitige Orientierung zwischen Hochfrequenzspule und Röntgenquelle dann auch bei einer Drehung des aus Röntgenquelle und Röntgendetektor gebildeten Röntgensystems um die Drehachse konstant. Dadurch werden Beeinflussungen des Röntgensystems durch die Hochfrequenzspule minimiert. Eine durch die Hochfrequenzspule bewirkte Absorption von durch die Röntgenquelle ausgesandter Röntgenstrahlung ist zeitlich unveränderlich. Dies ermöglicht es vorteilhafterweise, durch die Hochfrequenzspule bewirkte Artefakte in durch das Röntgensystem erstellten Aufnahmen zu minimieren oder zu entfernen.

In einer Ausführungsform des Untersuchungssystems sind eine zweite Röntgenquelle und ein zweiter Röntgendetektor im Untersuchungsraum angeordnet. Das Vorsehen zweier Röntgensysteme ermöglicht vorteilhafterweise eine erhöhte Aufnahmegeschwindigkeit von Röntgenbildern, eine Möglichkeit zur Reduzierung einer Röntgendosis und eine Möglichkeit zur gleichzeitigen Verwendung von Röntgenstrahlen unterschiedlicher Energien. Dadurch können zusätzliche Informationen über ein Durchblutungsverhalten und einen Typ und eine Zusammensetzung eies untersuchten Gewebes gewonnen werden.

In einer Ausführungsform des Untersuchungssystems sind die erste Röntgenquelle und die zweite Röntgenquelle starr miteinander verbunden. Vorteilhafterweise ist eine Blickwinkeldifferenz zwischen der ersten Röntgenquelle und der zweiten Röntgenquelle dann zeitlich konstant.

In einer Ausführungsform des Untersuchungssystems ist die zweite Röntgenquelle bezüglich einer Drehung um eine in Längsrichtung des Untersuchungsraums angeordnete Drehachse um einen Winkel gegen die erste Röntgenquelle versetzt. Vorteilhafterweise kann ein zu untersuchender Körperteil eines Patienten mittels der beiden Röntgensysteme dann aus zwei Richtungen gleichzeitig durchleuchtet werden.

In einer Ausführungsform des Untersuchungssystems beträgt der Winkel 90 Grad. Vorteilhafterweise lassen sich die mittels der beiden Röntgensysteme generierten Röntgenbilder dann besonders einfach miteinander korrelieren.

Bei einem Verfahren zum Herstellen eines Angiogramms wird ein Untersuchungssystem der vorgenannten Art verwendet. Vorteilhafterweise kann das Angiogramm dann mittels einer magnetresonanztomographischen Untersuchung gewonnene Informationen und mittels einer Röntgenuntersuchung gewonnene Informationen umfassen, wodurch das nach diesem Verfahren hergestellte Angiogramm einen besonders hohen Informationsgehalt aufweist. Die oben beschriebenen Eigenschaften, Merkmale und Vorteile dieser Erfindung, sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden. Hierbei zeigen in jeweils schematischer Darstellung:
Figur 1 eine geschnittene perspektivische Darstellung eines Untersuchungssystems gemäß eines ersten Beispiels, das nicht in den Schutzumfang der Erfindung fällt;
Figur 2 eine geschnittene perspektivische Darstellung eines Untersuchungssystems gemäß einer Ausführungsform;
Figur 3 eine geschnittene perspektivische Darstellung eines Untersuchungssystems gemäß eines zweiten Beispiels, das nicht in den Schutzumfang der Erfindung fällt;
Figur 4 eine weitere geschnittene Darstellung des Untersuchungssystems gemäß des zweiten Beispiels.

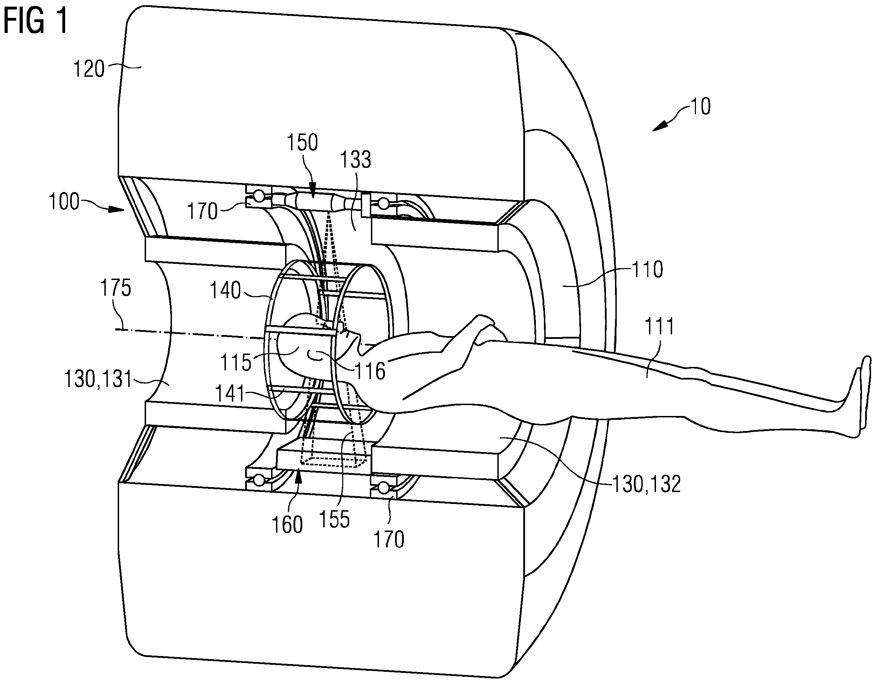

Figur 1 zeigt eine schematische geschnittene perspektivische Darstellung eines Untersuchungssystems 10 gemäß eines ersten Beispiels, das nicht in den Schutzumfang der Erfindung fällt. Untersuchungssystem 10 kann zur medizinischen Diagnostik dienen. Insbesondere kann das Untersuchungssystem 10 zur behandelnden Angiographie dienen.

Das Untersuchungssystem 10 umfasst einen Magnetresonanztomographen 100. Der Magnetresonanztomograph 100 weist eine Zylinderspule 120 auf, die einen röhrenförmigen Untersuchungsraum 110 umgibt. Die Zylinderspule 120 ist als langgestreckter Hohlzylinder ausgebildet. Der zylindrische Untersuchungsraum 110 dient zur Aufnahme eines schematisch dargestellten Patienten 111. Im dargestellten Beispiel ist in einem Sichtfeld 115 im Untersuchungsraum 110 des Untersuchungssystems 10 ein Körperteil 116 (hier der Kopf) des Patienten 111 angeordnet, um mit Hilfe des Untersuchungssystems 10 zur medizinischen Diagnostik geeignete Bilder des Körperteils 116 des Patienten 111 zu gewinnen.

Die Zylinderspule 120 des Magnetresonanztomographen 100 dient zur Erzeugung eines starken statischen magnetischen Feldes im Untersuchungsraum 110, durch das eine energetische Entartung zwischen unterschiedlichen Ausrichtungen der magnetischen Momente der Atomkerne des Körpers des Patienten 111 aufgehoben wird. Die Zylinderspule 120 kann beispielsweise als supraleitender Magnet ausgebildet sein. Die Zylinderspule 120 kann beispielsweise dazu ausgebildet sein, ein statisches Magnetfeld einer Stärke zwischen 0,2 Tesla und 3 Tesla oder mehr zu erzeugen. Das durch die Zylinderspule 120 erzeugte Magnetfeld ist zumindest im Sichtfeld 115 des Untersuchungsraums 110 bevorzugt etwa homogen ausgebildet. Unter dem Einfluss des durch die Zylinderspule 120 erzeugten statischen Magnetfelds präzedieren die Kernspins der Atomkerne des Körpers des Patienten 111 um die durch das Magnetfeld vorgegebene Achse (Larmor-Präzesion) .

Zur Erzeugung von Schichtbildern des im Sichtfeld 115 des Untersuchungsraums 110 angeordneten Körperteils 116 des Patienten 111 ist es notwendig, das durch die Zylinderspule 120 erzeugte homogene Magnetfeld ortsabhängig zu modifizieren. Hierzu verfügt der Magnetresonanztomograph 100 über eine Gradientenspule 130. Die Gradientenspule 130 ist dazu vorgesehen, während einer durch den Magnetresonanztomographen 100 durchgeführten Messung ein magnetisches Gradientenfeld zur selektiven Schichtanregung und zur Ortscodierung eines Messsignals zu erzeugen.

Die Gradientenspule 130 umfasst einen ersten Gradientenspulenteil 131 und einen zweiten Gradientenspulenteil 132. Der erste Gradientenspulenteil 131 und der zweite Gradientenspulenteil 132 sind jeweils hohlzylindrisch ausgebildet und koaxial zur Zylinderspule 120 im von der Zylinderspule 120 umgebenen Untersuchungsraum 110 angeordnet. Dabei sind der erste Gradientenspulenteil 131 und der zweite Gradientenspulenteil 132 der Gradientenspule 130 in axiale Richtung voneinander beabstandet, so dass zwischen dem ersten Gradientenspulenteil 131 und dem zweiten Gradientenspulenteil 132 ein Zwischenraum 133 ausgebildet ist. Im Bereich dieses Zwischenraums 132 befindet sich das Sichtfeld 115. Im dargestellten Beispiel weist der erste Gradientenspulenteil 131 einen geringeren Durchmesser als der zweite Gradientenspulenteil 132 auf. Dies ist jedoch nicht zwingend erforderlich.

Durch Einstrahlen eines senkrecht zum durch die Zylinderspule 120 erzeugten statischen Magnetfeld orientierten magnetischen Wechselfeldes mit einer Resonanzfrequenz können die um die Achse des statischen Magnetfelds präzidierenden Kernspins der Atome des Körpers des Patienten 111 phasensynchron ausgelenkt (angeregt) werden. Hierzu weist der Magnetresonanztomograph 100 eine Hochfrequenzspule 140 auf. Die Hochfrequenzspule 140 ist im Zwischenraum 133 zwischen dem ersten Gradientenspulenteil 131 und dem zweiten Gradientenspulenteil 132 der Gradientenspule 130 angeordnet. Die Hochfrequenzspule 140 umfasst zwei koaxial zur Zylinderspule 120 angeordnete Ringe, die über eine Mehrzahl von Sprossen 141 miteinander verbunden sind. Die Hochfrequenzspule 140 ist um eine Drehachse 175 drehbar, die einer Längsachse der Zylinderspule 120 und des Untersuchungsraums 110 entspricht. Das Sichtfeld 115 des Untersuchungsraums 110 ist innerhalb der Hochfrequenzspule 140 angeordnet.

Die Hochfrequenzspule 140 eignet sich zum Einstrahlen hochfrequenter magnetischer Impulse. Nach dem Abschalten eines durch die Hochfrequenzspule 140 eingestrahlten magnetischen Wechselfeldes relaxieren die durch das magnetische Wechselfeld angeregten Kernspins nach charakteristischer Relaxationszeit und senden dabei Signale aus, die durch die Hochfrequenzspule 140 empfangen werden. Aus den durch die Hochfrequenzspule 140 empfangenen Signaldaten lässt sich durch mathematische Verfahren ein Abbild des im Sichtfeld 115 angeordneten Körperteils 116 des Patienten 111 rekonstruieren.

Das Untersuchungssystem 10 umfasst ferner ein Röntgensystem mit einer Röntgenquelle 150 und einem Röntgendetektor 160. Die Röntgenquelle 150 und der Röntgendetektor 160 sind im Zwischenraum 133 zwischen dem ersten Gradientenspulenteil 131 und dem zweiten Gradientenspulenteil 132 der Gradientenspule 130 im Untersuchungsraum 110 angeordnet. Die Röntgenquelle 150 ist dazu ausgebildet, einen Röntgenstrahl 155 auszusenden, der durch das Sichtfeld 115 verläuft und nach dem Durchlaufen des Sichtfelds 115 auf den Röntgendetektor 160 trifft. Das aus Röntgenquelle 150 und Röntgendetektor 160 gebildete Röntgensystem erlaubt eine schnelle Erstellung von Röntgenbildern des im Sichtfeld 115 angeordneten Körperteils 116 des Patienten 111 mit hoher zeitlicher Auflösung.

Die Röntgenquelle 150 und der Röntgendetektor 160 sind mittels eines Lagers 170 drehbar um die Drehachse 175 gelagert und starr miteinander verbunden. Dabei besteht zwischen der Röntgenquelle 150 und dem Röntgendetektor 160 bezüglich einer Drehung um die Drehachse 175 eine Phasendifferenz von etwa 180 Grad. Die um die Drehachse 175 drehbare Anordnung des aus Röntgenquelle 150 und Röntgendetektor 160 gebildeten Röntgensystems erlaubt es, den im Sichtfeld 115 des Untersuchungsraums 110 angeordneten Körperteil 116 des Patienten 111 aus unterschiedlichen Blickrichtungen zu durchstrahlen und damit Durchsichten des Körperteils 116 aus unterschiedlichen Blickrichtungen zu erstellen.

Durch die gemeinsame Anordnung der Hochfrequenzspule 140 des Magnetresonanztomographen 100 und des aus Röntgenquelle 150 und Röntgendetektor 160 gebildeten Röntgensystems im Zwischenraum 133 um das Sichtfeld 115 ist sichergestellt, dass die mit den Methoden der Magnetresonanztomographie durch den Magnetresonanztomographen 100 und mit den Methoden der Röntgenuntersuchung durch das Röntgensystem gewonnenen anatomischen Informationen zeitlich und räumlich konsistent sind. Die mit beiden Untersuchungsmodalitäten gewonnenen Informationen ergänzen sich dabei.

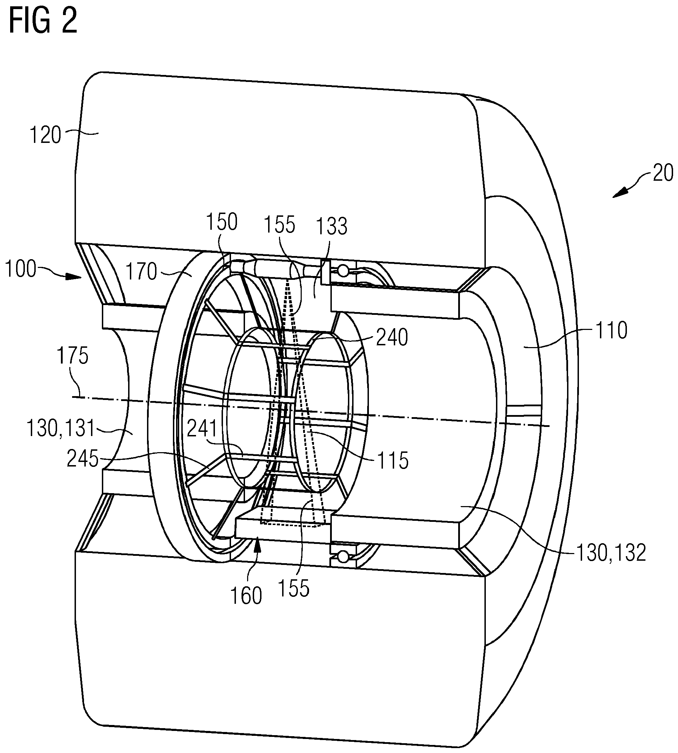

Figur 2 zeigt in schematischer perspektivischer Schnittdarstellung ein Untersuchungssystem 20 gemäß einer Ausführungsform. Das Untersuchungssystem 20 weist große Übereinstimmungen mit dem Untersuchungssystem 10 der Figur 1 auf. Entsprechende Komponenten sind daher mit denselben Bezugszeichen versehen und werden nachfolgend nicht erneuert detailliert beschrieben.

Anstelle der Hochfrequenzspule 140 ist beim Untersuchungssystem 20 eine Hochfrequenzspule 240 vorgesehen und im Zwischenraum 133 zwischen dem ersten Gradientenspulenteil 131 und dem Gradientenspulenteil 132 der Gradientenspule 130 um das Sichtfeld 115 im Untersuchungsraum 110 angeordnet. Die Hochfrequenzspule 240 weist wiederum zwei koaxial zur Zylinderspule 120 angeordnete Ringe auf, die mittels einer Mehrzahl von Sprossen miteinander verbunden sind.

Im Unterschied zur Hochfrequenzspule 140 ist die Hochfrequenzspule 240 allerdings mittels einer starren Verbindung 245 starr mit durch Röntgenquelle 150 und Röntgendetektor 160 gebildeten Röntgensystems verbunden. Die Hochfrequenzspule 240 ist daher gemeinsam mit dem Röntgensystem mittels des Lagers 170 drehbar um die Drehachse 175 gelagert. Die Hochfrequenzspule 240 ist dadurch phasensynchron zum aus Röntgenquelle 150 und Röntgendetektor 160 gebildeten Röntgensystem um die Drehachse 175 drehbar. Während einer Drehung der Hochfrequenzspule 240 und des Röntgensystems um die Drehachse 175 bleibt die relative Anordnung zwischen der Hochfrequenzspule 240, der Röntgenquelle 150 und dem Röntgendetektor 160 konstant.

Dies bietet den Vorteil, dass durch die Hochfrequenzspule 240 bedingte Einflüsse auf mittels des Röntgensystems aufgenommene Röntgenbilder zeitlich konstant sind und minimiert werden können. Da die Röntgenquelle 150, der Röntgendetektor 160 und die Hochfrequenzspule 240 starr zueinander angeordnet sind, ist eine durch die Hochfrequenzspule 240 bewirkte Absorption des Röntgenstrahls 155 zeitlich invariant. Dies ermöglicht es, eventuelle Artefakte in mittels des Röntgensystems erstellten Röntgenbildern, beispielsweise durch die Sprossen 241 der Hochfrequenzspule 240 bewirkte Artefakte, zu minimieren oder zu entfernen. Beispielsweise können die Hochfrequenzspule 240, die Röntgenquelle 150 und der Röntgendetektor 160 zueinander so orientiert sein, dass der Röntgenstrahl 155 nicht auf eine der Sprossen 241 der Hochfrequenzspule 240 trifft. Ebenfalls ist es möglich, zeitlich konstant auftretende Artefakte mit den Methoden der digitalen Bildverarbeitung herauszurechnen.

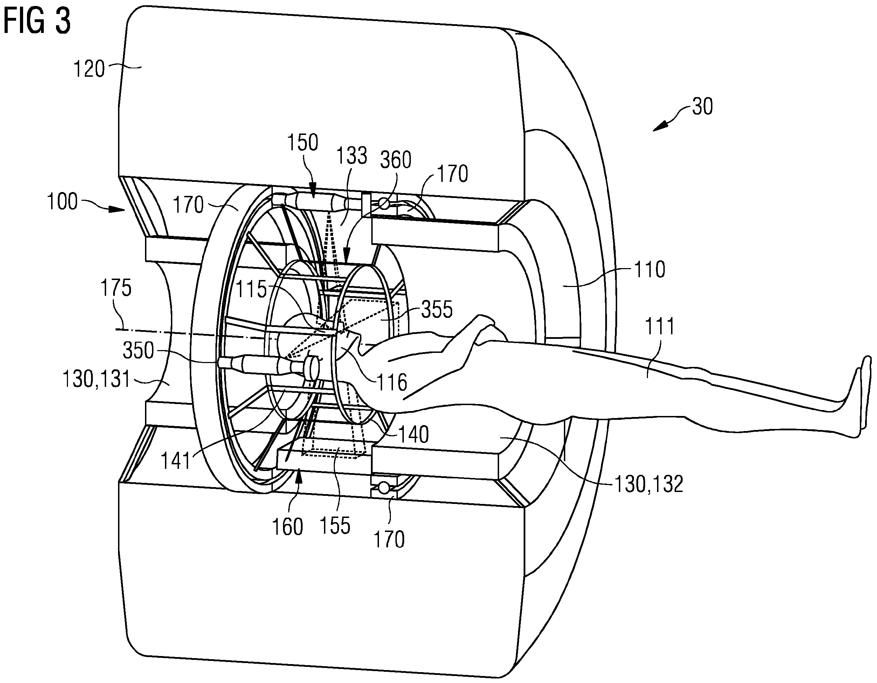

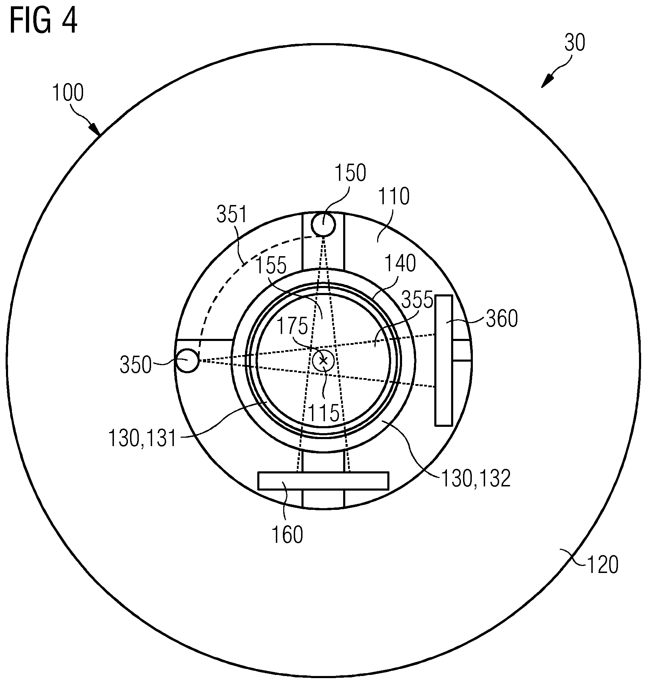

Figur 3 zeigt eine schematische perspektivische Schnittdarstellung eines Untersuchungssystems 30 gemäß eines zweiten Beispiels, das nicht in den Schutzumfang der Erfindung fällt. Figur 4 zeigt eine weitere Schnittdarstellung des Untersuchungssystems 30. In der Darstellung der Figur 4 verläuft der Schnitt senkrecht zur Drehachse 175. Das Untersuchungssystem 30 weist Übereinstimmungen mit dem Untersuchungssystem 10 der Figur 1 auf. Einander entsprechende Komponenten sind daher mit denselben Bezugszeichen versehen und werden nachfolgend nicht erneut detailliert beschrieben.

Zusätzlich zu den Komponenten des Untersuchungssystems 10 sind beim Untersuchungssystem 30 eine zweite Röntgenquelle 350 und ein zweiter Röntgendetektor 360 vorhanden. Die zweite Röntgenquelle 350 und der zweite Röntgendetektor 360 bilden ein zweites Röntgensystem. Die zweite Röntgenquelle 350 und der zweite Röntgendetektor 360 sind ebenfalls im Zwischenraum 133 zwischen dem ersten Gradientenspulenteil 131 und dem zweiten Gradientenspulenteil 132 der Gradientenspule 130 angeordnet und so orientiert, dass ein durch die zweite Röntgenquelle 350 ausgesandter zweiter Röntgenstrahl 355 durch das Sichtfeld 115 verläuft, bevor er auf den zweiten Röntgendetektor 360 trifft.

Die zweite Röntgenquelle 350 und der zweite Röntgendetektor 360 sind ebenfalls mittels des Lagers 170 drehbar um die Drehachse 375 gelagert. Die zweite Röntgenquelle 350 und der zweite Röntgendetektor 360 sind derart starr miteinander verbunden, dass zwischen der zweiten Röntgenquelle 350 und dem zweiten Röntgendetektor 360 bezüglich einer Drehung um die Drehachse 175 ein konstantes Winkelverhältnis von bevorzugt etwa 180 Grad besteht.

Bevorzugt sind auch die Röntgenquelle 150 und die zweite Röntgenquelle 350 derart starr miteinander verbunden, dass zwischen der Röntgenquelle 150 und der zweiten Röntgenquelle 350 bezüglich einer Drehung um die Drehachse 175 stets ein konstanter Winkel 351 besteht. Der Winkel 351 kann zwischen 0 Grad und 180 Grad betragen. Bevorzugt beträgt der Winkel 351 etwa 90 Grad. Mittels des aus der Röntgenquelle 150 und dem Röntgendetektor 160 gebildeten Röntgensystems aufgenommene Röntgenbilder und mittels des durch die zweite Röntgenquelle 350 und den zweiten Röntgendetektor 360 gebildeten zweiten Röntgensystems aufgenommene Röntgenbilder zeigen den im Sichtfeld 115 angeordneten Körperteil 116 des Patienten 111 dann aus zueinander senkrechten Blickrichtungen.

Das Vorhandensein zweier Röntgensysteme beim Untersuchungssystem 30 bietet den Vorteil, dass Röntgenbilder mit noch höherer Geschwindigkeit und Bildwiederholfrequenz aufnehmbar sind. Außerdem eröffnet sich dadurch die Möglichkeit, eine Röntgendosis zu reduzieren. Die beiden Röntgensysteme können auch bei unterschiedlichen Wellenlängen bzw. Energieniveaus betrieben werden. Dadurch lassen sich zusätzliche Informationen über den im Sichtfeld 115 angeordneten Körperteil 116 des Patienten 111 gewinnen. Beispielsweise lassen sich dadurch zusätzliche Informationen über ein Durchblutungsverhalten oder über Gewebearten des Körperteils 116 gewinnen.

Bei den Untersuchungssystemen 10, 20, 30 werden der Magnetresonanztomograph 100 und die Röntgensysteme bevorzugt jeweils gleichzeitig betrieben. Es ist jedoch auch möglich, für einzelne Untersuchungen nur entweder den Magnetresonanztomographen oder das Röntgensystem zu betreiben. Die beiden Untersuchungsmodalitäten können auch zeitlich nacheinander erfolgen.

Obwohl die Erfindung im Detail durch das bevorzugte Ausführungsbeispiel näher illustriert und beschrieben wurde, ist die Erfindung nicht durch die offenbarten Beispiele eingeschränkt. Andere Variationen können vom Fachmann hieraus abgeleitet werden, ohne den Schutzumfang der Erfindung zu verlassen, der durch die nachfolgenden Ansprüche definiert wird.

## Patentansprüche

1. Untersuchungssystem (10, 20, 30)
mit einem Magnetresonanztomographen (100) mit einer Zylinderspule (120) zur Erzeugung eines Magnetfelds,
wobei die Zylinderspule (120) einen Untersuchungsraum (110) umgibt,
wobei im Untersuchungsraum (110) eine erste Röntgenquelle (150) und ein erster Röntgendetektor (160) angeordnet sind und
wobei eine Hochfrequenzspule (140, 240) im Untersuchungsraum (110) angeordnet ist,
**dadurch gekennzeichnet, dass**
die Hochfrequenzspule (240) starr mit der ersten Röntgenquelle (150) verbunden ist.

2. Untersuchungssystem (10, 20, 30) gemäß Anspruch 1,
wobei die erste Röntgenquelle (150) und der erste Röntgendetektor (160) um eine in Längsrichtung des Untersuchungsraums (110) angeordnete Drehachse (175) drehbar sind.

3. Untersuchungssystem (10, 20, 30) gemäß einem der vorhergehenden Ansprüche,
wobei die erste Röntgenquelle (150) und der erste Röntgendetektor (160) starr miteinander verbunden sind.

4. Untersuchungssystem (10, 20, 30) gemäß einem der vorhergehenden Ansprüche,
wobei im Untersuchungsraum (110) eine Gradientenspule (130) mit einem ersten Gradientenspulenteil (131) und einem zweiten Gradientenspulenteil (132) angeordnet ist, wobei der erste Gradientenspulenteil (131) und der zweite Gradientenspulenteil (132) in axialer Richtung beabstandet sind,
wobei die erste Röntgenquelle (150) und der erste Röntgendetektor (160) zwischen dem ersten Gradientenspulenteil (131) und dem zweiten Gradientenspulenteil (132) angeordnet sind.

5. Untersuchungssystem (10, 20, 30) gemäß einem der vorhergehenden Ansprüche,
wobei die Hochfrequenzspule (140, 240) zwischen dem ersten Gradientenspulenteil (131) und dem zweiten Gradientenspulenteil (132) angeordnet ist.

6. Untersuchungssystem (30) gemäß einem der vorhergehenden Ansprüche,
wobei eine zweite Röntgenquelle (350) und ein zweiter Röntgendetektor (360) im Untersuchungsraum (110) angeordnet sind.

7. Untersuchungssystem (30) gemäß Anspruch 6,
wobei die erste Röntgenquelle (150) und die zweite Röntgenquelle (350) starr miteinander verbunden sind.

8. Untersuchungssystem (30) gemäß Anspruch 7,
wobei die zweite Röntgenquelle (350) bezüglich einer Drehung um eine in Längsrichtung des Untersuchungsraums (110) angeordnete Drehachse (175) um einen Winkel (351) gegen die erste Röntgenquelle (150) versetzt ist.

9. Untersuchungssystem (30) gemäß Anspruch 8,
wobei der Winkel (351) 90 Grad beträgt.

10. Verfahren zum Herstellen eines Angiogramms,
wobei ein Untersuchungssystem (10, 20, 30) gemäß einem der vorhergehenden Ansprüche verwendet wird.

## Claims

1. Examination system (10, 20, 30) having
a magnetic resonance scanner (100) with a cylindrical coil (120) for generating a magnetic field,
wherein the cylindrical coil (120) surrounds an examination space (110),
wherein a first X-ray source (150) and a first X-ray detector (160) are arranged in the examination space (110) and
wherein a radiofrequency coil (140, 240) is arranged in the examination space (110),
**characterized in that**
the radiofrequency coil (240) is rigidly connected to the first X-ray source (150).

2. Examination system (10, 20, 30) according to Claim 1,
wherein the first X-ray source (150) and the first X-ray detector (160) are rotatable about a rotation axis (175) arranged in a longitudinal direction of the examination space (110).

3. Examination system (10, 20, 30) according to any one of the preceding claims,
wherein the first X-ray source (150) and the first X-ray detector (160) are rigidly interconnected.

4. Examination system (10, 20, 30) according to any one of the preceding claims,
wherein a gradient coil (130) with a first gradient coil portion (131) and a second gradient coil portion (132) is arranged in the examination space (110),
wherein the first gradient coil portion (131) and the second gradient coil portion (132) are spaced apart in an axial direction, and
wherein the first X-ray source (150) and the first X-ray detector (160) are arranged between the first gradient coil portion (131) and the second gradient coil portion (132).

5. Examination system (10, 20, 30) according to any one of the preceding claims,
wherein the radiofrequency coil (140, 240) is arranged between the first gradient coil portion (131) and the second gradient coil portion (132).

6. Examination system (30) according to any one of the preceding claims,
wherein a second X-ray source (350) and a second X-ray detector (360) are arranged in the examination space (110).

7. Examination system (30) according to Claim 6,
wherein the first X-ray source (150) and the second X-ray source (350) are rigidly interconnected.

8. Examination system (30) according to Claim 7,
wherein the second X-ray source (350) is offset from the first X-ray source (150) by an angle (351) with reference to a rotation about a rotation axis (175) arranged in a longitudinal direction of the examination space (110).

9. Examination system (30) according to Claim 8,
wherein the angle (351) is 90 degrees.

10. Method for producing an angiogram,
wherein an examination system (10, 20, 30) according to any one of the preceding claims is used.

## Revendications

1. Système (10, 20, 30) d'examen,
comprenant un tomodensitomètre (100) à résonance magnétique ayant une bobine (120) cylindrique de production d'un champ magnétique, la bobine (120) cylindrique entourant un espace (110) d'examen,
dans lequel une première source (150) de rayons X et un premier détecteur (160) de rayons X sont disposés dans l'espace (110) d'examen et
dans lequel une bobine (140, 240) de haute fréquence est disposée dans l'espace (110) d'examen,
**caractérisé en ce que**
la bobine (240) de haute fréquence est reliée fixement à la première source (150) de rayons X.

2. Système (10, 20, 30) d'examen suivant la revendication 1,
dans lequel la première source (150) de rayons X et le premier détecteur (160) de rayons X peuvent tourner autour d'un axe (175) de rotation disposé dans la direction longitudinale de l'espace (110) d'examen.

3. Système (10, 20, 30) d'examen suivant l'une des revendications précédentes,
dans lequel la première source (150) de rayons X et le premier détecteur (160) de rayons X sont reliés fixement l'un à l'autre.

4. Système (10, 20, 30) d'examen suivant l'une des revendications précédentes,
dans lequel, dans l'espace (110) d'examen, est disposée une bobine (130) de gradient ayant une première partie (131) de bobine de gradient et une deuxième partie (132) de bobine de gradient, la première partie (131) de bobine de gradient et la deuxième partie (132) de bobine de gradient étant à distance dans la direction axiale,
dans lequel la première source (150) de rayons X et le premier détecteur (160) de rayons X sont disposés entre la première partie (131) de bobine de gradient et la deuxième partie (132) de bobine de gradient.

5. Système (10, 20, 30) d'examen suivant l'une des revendications précédentes,
dans lequel la bobine (140, 240) de haute fréquence est disposée entre la première partie (131) de bobine de gradient et la deuxième partie (132) de bobine de gradient.

6. Système (30) d'examen suivant l'une des revendications précédentes,
dans lequel une deuxième source (350) de rayons X et un deuxième détecteur (360) de rayons X sont disposés dans l'espace (110) d'examen.

7. Système (30) d'examen suivant la revendication 6,
dans lequel la première source (150) de rayons X et la deuxième source (350) de rayons X sont reliées fixement l'une à l'autre.

8. Système (30) d'examen suivant la revendication 7,
dans lequel la deuxième source (350) de rayons X est, rapporté à une rotation autour d'un axe (175) de rotation disposé dans la direction longitudinale de l'espace (110) d'examen, décalée d'un angle (351) par rapport à la première source (150) de rayons X.

9. Système (30) d'examen suivant la revendication 8,
dans lequel l'angle (351) est de 90 degrés.

10. Procédé de production d'un angiogramme,
dans lequel on utilise un système (10, 20, 30) d'examen suivant l'une des revendications précédentes.
